# EUROPÄISCHE PATENTSCHRIFT

(11) **EP 1 879 609 B1**
(45) Veröffentlichungstag und Bekanntmachung des Hinweises auf die Patenterteilung: **12.03.2014**
(21) Anmeldenummer: 06742792.2
(22) Anmeldetag: 04.05.2006
(51) Int. Cl.: A61K 38/17, A61P 7/00

(54) **VERWENDUNG VON HISTONEN ZU THERAPEUTISCHEN ZWECKEN**
USE OF HISTONES FOR THERAPEUTIC PURPOSES
UTILISATION D'HISTONES A DES FINS THERAPEUTIQUES

(30) Priorität: 10.05.2005 DE 102005022319
(43) Veröffentlichungstag der Anmeldung: 23.01.2008
(73) Patentinhaber: SYMBIOTEC Gesellschaft zur Forschung und Entwicklung auf dem Gebiet der Biotechnologie mbH, 66123 Saarbrücken (DE)
(72) Erfinder: ZEPPEZAUER, Michael, 66133 Scheidt/Saarbrücken (DE); REINER, Class, 66740 Saarlouis (DE)
(74) Vertreter: Schlich, George
(86) Internationale Anmeldenummer: PCT/EP2006/004167
(87) Internationale Veröffentlichungsnummer: WO 2006/119912

(56) Entgegenhaltungen:
- DE-A1- 19 715 149

## Beschreibung

Die Erfindung betrifft die Verwendung wenigstens eines humanen rekombinanten Histon H1-Subtyps und/oder seines therapeutisch wirksamen Abschnittes, insbesondere Histon H1.3 zu therapeutischen Zwecken.

Die Verwendung von therapeutischen Wirkstoffen auf der Basis humaner rekombinanter Histone H1-Subtypen zur Behandlung von Krebserkrankungen, z.B. Leukämie, ist durch den Artikel von Reiner Class et al. in Am. J. Clin. Oncol. (CCT) Vol. 19 No. 5 1996 und die Europäische Patentanmeldung 98919254.7 bekannt.

Die Winkung von Histon H1 und H2A / H2B Fraktionen aus Kalbsthymus auf hämatopoetische Stammzellen (CFU-S) in normalen und radioaktiv bestrahlten Ratten wurde in einem russischen Artikel von Semina O.V. et al. in Radiatsionnaia Bioiogiia, Radioecologiia 34(4-5), 1994 Jul.-Oct. beschrieben.

Bei komplexen Krankheitsbildern, wie der akuten myeloischen Leukämie, wird häufig eine Thrombozytopenie beobachtet, die durch eine chemotherapeutische Behandlung der Leukämie noch verstärkt werden kann. Thrombozytopenie wird aber auch bei anderer Ätiologie beobachtet. Da Thrombozytopenie zu lebensbedrohlichen inneren Blutungen führen kann, sind Therapien gegen unterschiedlichste Krankheitssymptome, die Ursache der Thrombozytopenie sein können, häufig besonders erschwert.

Aufgabe der Erfindung war es daher, einen therapeutisch verwendbaren Wirkstoff gegen Thrombozytopenie zu finden, um damit auch die Heilungserfolge des Grundsymptoms als Auslöser der Thrombozytopenie wesentlich zu verbessern. Die Aufgabe konnte erfindungsgemäß dadurch gelöst werden, dass zur Therapie von Thrombozytopenie unter anderem infolge fehlerhafter Stammzellen-Differentiation oder geschwächter Proliferation der Megakaryocyten ein Wirkstoff auf der Basis wenigstens eines humanen rekombinanten Histons (insbesondere wenigstens eines Histon H1-Subtyps) und/oder seines therapeutisch wirksamen Abschnittes verwendet wird.

Das gilt insbesondere für eine Thrombozytopenie als Begleiterscheinung einer hämatologischen Erkrankung.

Dabei kann das efindungsgemäße therapeutische Verfahren zur Behandlung der Thrombozytopenie während oder nach einer Chemotherapie zur Behandlung einer hämatologischen Erkrankung, insbesondere der akuten myeloischen Leukämie angewendet werden.

Uberraschenderweise konnte gezeigt werden, dass der erfindungsgemäße Wirkstoff einerseits ein positives Ergebnis bei der Behandlung einer hämatologischen Erkrankung, wie der Leukämie, andererseits aber auch ein positives Ergebnis bei der Behandlung der mit den hämatologischen Erkrankungen einhergehenden Thrombozytopenie zeigt.

Mit ein- und demselben Wirkstoff konnte sowohl ein Rückgang der Leukämie als auch eine Erhöhung der Thrombozyten verzeichnet werden.

Therapeutische Versuchsergebnisse auch an Patienten konnten mit humanen rekombinanten Histon H1.3 erzielt werden, wobei die Anzahl der pathologischen Tumorzellen in einem AML-Patienten deutlich gesenkt und gleichzeitig die Thrombozytenproduktion wesentlich erhöht werden konnte, wodurch die Heilungschancen des Patienten wesentlich verbessert werden konnten.

Die Versuchsergebnisse sind nachstehend mehr im einzelnen wiedergegeben, wobei der hierbei verwendete Wirkstoff auf der Basis von humanen rekombinanten H1.3 Konzentration 37,5 mg/qm² Körperoberfläche usw. in einer 0,9% NaCl-Lösung intravenös 3 mal pro Woche über eine Dauer von ca. 4 Stunden verabreicht wurde.

Die Wirkung des erfindungsgemäßen Wirkstoffes in einer 0,9% NaCl-Lösung bei 37,5 mg/qm² Körperoberfläche bei der Thrombozytopenie eines Patienten ist in dem anliegenden Diagram gezeigt.

Auf der Ordinate sind die Thrombozyten im peripheren Blut in einer Anzahl von 0 bis 40 × 10⁹. Auf der Abszisse ist eine dreiwöchige Behandlung mit efindungsgemäßem Wirkstoff gezeigt, wobei bei dem Patienten vor der ersten Behandlung eine stark verringerte Thrombozytenzahl von etwa 8 × 10⁹ gemessen wurde. Es erfolgen dann jeweils drei Tropfinfusionen pro Woche von jeweils 4 Stunden pro Infusion mit der 1. bis 3. Infusion am 1., 3. und 5. Tag in der ersten Woche, mit der 4. bis 6. Infusion am 8., 10. und 12. Tag in der zweiten Woche und abschließend mit der 7. bis 9. Infusion am 15., 17., und 19. Tag in der 3. Woche. Am 29. Tag nach der ersten Infusion fand eine Kontrollmessung der Thrombozytenzahl statt, ohne eine weitere Infusion mit dem erfindungsgemäßen Wirkstoff. Zu einem späteren Zeitpunkt FU1 erfolgte die Entlassung des Patienten mit einer fast normalen Thrombozytenzahl von etwa 34 × 10⁹, ohne dass noch einmal ein erfindungsgemäßer Wirkstoff zugeführt worden ist. Dieser Wert lag außerhalb der Notwendigkeit der Zufuhr von Blutkonserven. Der Patient wurde zu einer Nachuntersuchung bestellt mit der Möglichkeit einer erneuten Behandlungsaufnahme falls die Thrombozytenzahl sich nicht weiter von selbst verbessert hat oder sogar verschlechtert haben sollte. Die Nachuntersuchungsergebnisse sind hier nicht dargestellt.

Das anliegende Diagram zeigt zu Beginn der 2. Woche bis zum Abschluß der Behandlung in der dritten Woche einen sprunghaften Ansteig der Thrombozytenzahl nach dem 5. Behandlungstag und dann einen kontinuierlichen Anstieg der Thrombozytenzahl vom 8. bis zum anschließenden 19. Behandlungstag und einen weiteren verlangsamten Anstieg der Thrombozytenzahl bei der ersten Kontrolluntersuchung am 29. Tag und an einem späteren Entlassungstag FU1 des Patienten ohne weitere Zugabe des erfindungsgemäßen Wirkstoffes, wobei die zuletzt gemessene Thrombozytenzahl, wie schon gesagt, etwa 32,5 × 10⁹ betrug.

Die Erfindung ist nicht auf die Verwendung von humanen rekombinanten H1.3 beschränkt. Wegen der nahen Verwandtschaft der H1-Subtypen liegt es für den Fachmann nahe, als Wirkstoff auch andere humane rekombinante H1-Subtypen als Basis für den erfindungsgemäßen Wirkstoff einzusetzen.

Nach der erfolgreichen Therapie an Patienten mit einer Thrombozytopenie hier als Begleitsyndrom einer AML-Leukämie mit humanen rekombinanten Histon H1.3 liegt es für den Fachmann besonders nahe, auch andere rekombinanten H1-Subtypen als atternative Wirksubstanzen einzeln oder im Kombination erfindungsgemäß einzusetzen.

Vorzugsweise besteht der erfindungsgemäße Wirkstoff aus den vollständigen unverkürzten Subtypen von Histon-Proteinen. Für den Fachmann liegt es aber auch nahe, nach dem therapeutisch wirksamen Abschnitt zu suchen, wozu er aufgrund seiner Fachkenntnisse und Erfahrungen ohne weiteres in der Lage ist, ohne dass hierbei überragende innovative Leistungen zu erbringen sind. Solche therapeutisch wirksamen Histonabschnitte liegen daher im Äquivalenzbereich der hier offenbarten erfindungsgemäßen Lehre.

Weiterhin offenbart die Erfindung die therapeutische Lehre bei einer drohenden oder im Anfangszustand befindlichen primären Erkrankung, die erfahrungsgemäß eine Thrombozytopenie zur Folge haben kann, prophylaktisch den erfindungsgemäßen Wirkstoff gegen eine drohende Thrombozytopenie auch dann einzusetzen, wenn anders als bei der Leukämie der erfindungsgemäße Wirkstoff gegen die primäre Erkrankung nicht wirksam ist.

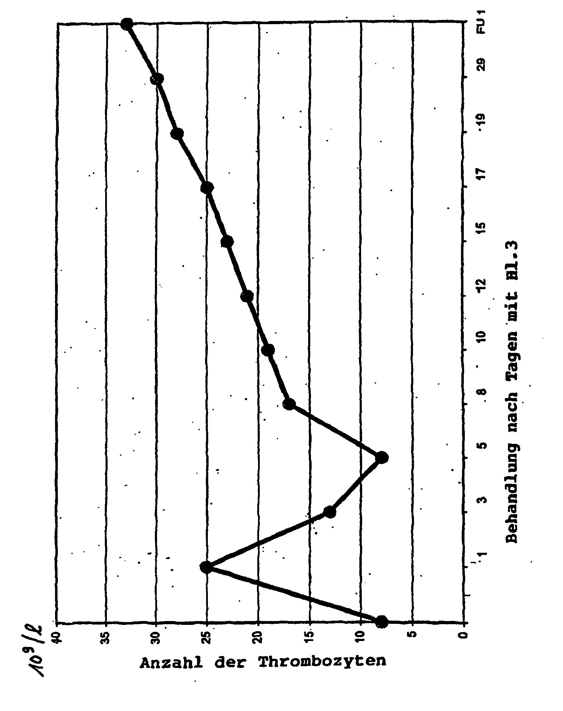

## Patentansprüche

1. Wenigstens ein humaner rekombinanter Histon H1-Subtyp, und/oder ein therapeutisch wirksamer Histonabschnitt desselben als Basis zur Verwendung in der therapeutischen Behandlung von Thrombozytopenie.

2. Wenigstens ein humaner rekombinanter Histon H1-Subtyp, und/oder ein therapeutisch wirksamer Histonabschnitt desselben zur Verwendung nach Anspruch 1 zur therapeutischen Behandlung von Thrombozytopenie als Begleiterscheinung hämatologischer Erkrankungen.

3. Wenigstens ein humaner rekombinanter Histon H1-Subtyp, und/oder ein therapeutisch wirksamer Histonabschnitt desselben zur Verwendung nach Anspruch 1 zur therapeutischen Behandlung von Thrombozytopenie während oder nach einer Therapie zur Behandlung hämatologischer Erkrankungen, insbesondere nach einer Chemotherapie.

4. Wenigstens ein humaner rekombinanter Histon H1-Subtyp, und/oder ein therapeutisch wirksamer Histonabschnitt desselben zur Verwendung nach Anspruch 1 zur therapeutischen Behandlung von Leukämie, insbesondere akuter myeloischer Leukämie bei gleichzeitiger therapeutischer Behandlung von Thromobozytopenie.

5. Wenigstens ein humaner rekombinanter Histon H1-Subtyp, und/oder ein therapeutisch wirksamer Histonabschnitt desselben zur Verwendung nach Anspruch 1 zur Therapie bei einer Thrombozytopenie infolge einer Erkrankung beliebiger Ätiologie.

6. Wenigstens ein humaner rekombinanter Histon H1-Subtyp, und/oder ein therapeutisch wirksamer Histonabschnitt desselben zur Verwendung nach Anspruch 5 zur prophylaktischen Therapie bei einer drohenden Thrombozytopenie infolge einer Erkrankung beliebiger Ätiologie.

7. Wenigstens ein humaner rekombinanter Histon H1-Subtyp, und/oder ein therapeutisch wirksamer Histonabschnitt desselben zur Verwendung nach Anspruch 1 zur therapeutischen Behandlung von Thrombozytopenie mittels humanen rekombinanten Histon H1.3 und/oder eines therapeutisch wirksamen Abschnittes von Histon H1.3.

## Claims

1. At least one human recombinant histone H1 subtype and/or a therapeutically effective histone segment thereof for use in the therapeutic treatment of thrombocytopenia.

2. At least one human recombinant histone H1 subtype and/or a therapeutically effective histone segment thereof for use according to claim 1 for the therapeutic treatment of thrombocytopenia as a concomitant symptom of haematological diseases.

3. At least one human recombinant histone H1 subtype and/or a therapeutically effective histone segment thereof for use according to claim 1 for the therapeutic treatment of thrombocytopenia during or after therapy for the treatment of haematological diseases, in particular after chemotherapy.

4. At least one human recombinant histone H1 subtype and/or a therapeutically effective histone segment thereof for use according to claim 1 for the therapeutic treatment of leukaemia, in particular acute myeloid leukaemia with the simultaneous therapeutic treatment of thrombocytopenia.

5. At least one human recombinant histone H1 subtype and/or a therapeutically effective histone segment thereof for use according to claim 1 for therapy in the case of thrombocytopenia resulting from a disease with any aetiology.

6. At least one human recombinant histone H1 subtype and/or a therapeutically effective histone segment thereof for use according to claim 5 for prophylactic therapy in the case of imminent thrombocytopenia resulting from a disease with any etiology.

7. At least one human recombinant histone H1 subtype and/or a therapeutically effective histone segment thereof for use according to claim 1 for the therapeutic treatment of thrombocytopenia using human recombinant histone H1.3 and/or a therapeutically effective segment of histone H1.3.

## Revendications

1. Au moins un sous-type H1 d'histone humaine recombinante et/ou un segment d'histone thérapeutiquement efficace de celui-ci comme base pour l'utilisation dans le traitement thérapeutique de la thrombocytopénie.

2. Au moins un sous-type H1 d'histone humaine recombinante et/ou un segment d'histone thérapeutiquement efficace de celui-ci pour l'utilisation selon la revendication 1, pour le traitement thérapeutique de la thrombocytopénie en tant que manifestation accompagnant des maladies hématologiques.

3. Au moins un sous-type H1 d'histone humaine recombinante et/ou un segment d'histone thérapeutiquement efficace de celui-ci pour l'utilisation selon la revendication 1, pour le traitement thérapeutique de la thrombocytopénie pendant ou après une thérapie pour le traitement de maladies hématologiques, en particulier après une chimiothérapie.

4. Au moins un sous-type H1 d'histone humaine recombinante et/ou un segment d'histone thérapeutiquement efficace de celui-ci pour l'utilisation selon la revendication 1, pour le traitement thérapeutique de la leucémie, en particulier de la leucémie myéloïde lors d'un traitement thérapeutique simultané de la thrombocytopénie.

5. Au moins un sous-type H1 d'histone humaine recombinante et/ou un segment d'histone thérapeutiquement efficace de celui-ci pour l'utilisation selon la revendication 1, pour la thérapie dans le cas d'une thrombocytopénie consécutive à une maladie d'étiologie quelconque.

6. Au moins un sous-type H1 d'histone humaine recombinante et/ou un segment d'histone thérapeutiquement efficace de celui-ci pour l'utilisation selon la revendication 5, pour la thérapie prophylactique dans le cas de thrombocytopénie imminente consécutive à une maladie d'étiologie quelconque.

7. Au moins un sous-type H1 d'histone humaine recombinante et/ou un segment d'histone thérapeutiquement efficace de celui-ci pour l'utilisation selon la revendication 1, pour le traitement thérapeutique de la thrombocytopénie au moyen de l'histone H1.3 humaine recombinante et/ou d'un segment thérapeutiquement efficace de l'histone H1.3.
